(19) Europäisches Patentamt
European Patent Office
Office européen des brevets

(11) Veröffentlichungsnummer: **0 422 623 A2**

(12) **EUROPÄISCHE PATENTANMELDUNG**

(21) Anmeldenummer: **90119431.6**

(51) Int. Cl.5: **C12M 1/40**

(22) Anmeldetag: **10.10.90**

(30) Priorität: **13.10.89 DE 3934299**

(43) Veröffentlichungstag der Anmeldung:
**17.04.91 Patentblatt 91/16**

(84) Benannte Vertragsstaaten:
**BE CH DE DK FR GB IT LI NL SE**

(71) Anmelder: **Gesellschaft für Biotechnologische Forschung mbH (GBF)**
**Mascheroder Weg 1**
**W-3300 Braunschweig-Stöckheim(DE)**

(72) Erfinder: **Schmid, Rolf, Prof. Dr.**
**Mascheroder Weg 1**
**W-3300 Braunschweig(DE)**
Erfinder: **Kulys, Juozas, Institute of Biochemistry**
**Lithuanian Academy of Sciences**
**Vilnius, 232021(SU)**

(74) Vertreter: **Boeters, Hans Dietrich, Dr. et al**
**Bereiteranger 15**
**W-8000 München 90(DE)**

(54) **Enzymelektrode und deren Verwendung.**

(57) Die Erfindung betrifft eine Enzymelektrode mit einem Bienzymsystem mit Pilzperoxidase oder Meerrettichperoxidase und einer oder mehreren Oxidasen sowie die Verwendung der Elektrode.

Fig.1

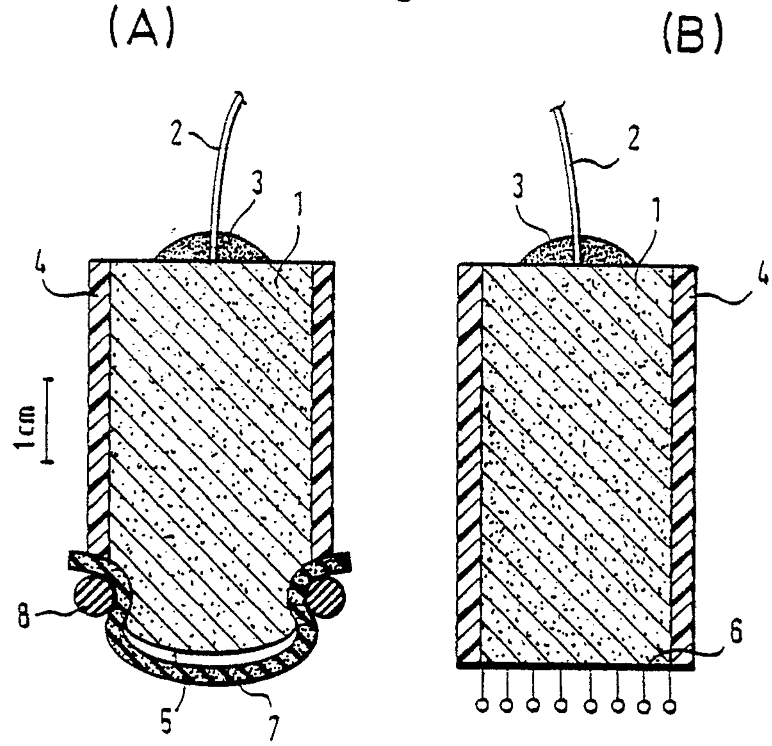


EP 0 422 623 A2

# ENZYMELEKTRODE UND DEREN VERWENDUNG

Eine Bienzymelektrode enthält eine Peroxidase und eine Oxidase. Sie ist generell von Nutzen in bioelektrochemischen Assays, z.B. zur Bestimmung der Substrate der Ethanoloxidase, Cholinoxidase, Cholesteroloxidase oder eine D-Aminosäurenoxidase.

## Hintergrund der Erfindung:

Enzymelektroden haben in Bezug auf Metabolitbestimmungen in Medizin, Biotechnologie, Lebensmitteltechnologie und vielen anderen Bereichen der Wissenschaft und Technologie beträchtliche Bedeutung erlangt (1). Zur Herstellung hochselektiver amperometrischer Enzymelektroden wurden Bienzymsysteme verwendet, die Peroxidasen und Oxidasen enthielten (2). Als löslicher Mediator wurde Ferrocyanid benutzt. Diese Elektroden waren für den Gebrauch in vivo oder die Kontrolle von Bioreaktoren nicht geeignet. Andere Systeme, die metallorganische Verbindungen und Peroxidase/Oxidase enthielten, kamen ohne Mediator aus, aber die Effizienz der Peroxidaseaktivität war niedrig, und die Elektroden zeigten ein verzögertes Ansprechen und ungenügende Stabilität.

Wir haben die Verwendung einer Peroxidase aus dem Pilz Arthromyces ramosus (3) oder chemisch modifizierter Elektroden (4) vorgeschlagen, um die Effizienz der Peroxidaseaktivität zu erhöhen und eine mediatorlose Bienzymelektrode herzustellen. Soweit uns bekannt ist, wurde zuvor noch keine Pilz-Peroxidase zur Herstellung eines elektrochemischen Systems verwendet, obwohl sie, in Abhängigkeit von dem benutzten Substrat, eine 2.9- bis 540-fach höhere katalytische Aktivität als Meerrettichperoxidase (5).

Chemische Modifikationen haben bei der Herstellung von Enzymelektroden breite Anwendung gefunden (6). Die erste chemisch modifizierte Elektrode enthielt Tetracyanochinodimethan und dessen Kaliumsalz (4). Leider reagieren manche Oxidasen wie z.B. Cholinoxidase, Cholesteroloxidase, Aminosäureoxidase und D-Aminosäureoxidase nicht mit Modifiziersubstanzen (7), so daß man keine einfach nur Oxidase enthaltende Elektrode konstruieren kann.

Gemäß einer Ausführungsform betrifft die Erfindung nun eine Enzymelektrode mit einem Bienzymsystem, das durch Pilzperoxidase und eine oder mehrere Oxidasen sowie Mediatorfreiheit gekennzeichnet ist.

Gemäß einer weiteren Ausführungsform betrifft die Erfindung eine Enzymelektrode mit einem Bienzymsystem, das durch Meerrettich-Peroxidase, eine oder mehrere Oxidasen und einen wasserunlöslichen Mediator gekennzeichnet ist.

Beispiel für wasserunlösliche Mediatoren sind Dimethylferrocen und Tetrathiafulvalen. Bei den Oxidasen kann es sich um Glukoseoxidase, Alkoholoxidase, Cholinoxidase, D-Aminosäureoxidase und/oder Cholesteroloxidase handeln.

Gemäß speziellen Ausführungsformen können

(a) die Peroxidase und die Oxidase(n) auf der Elektrode immobilisiert sein oder

(b) die Peroxidase auf der Elektrode immobilisiert sein und die Oxidase(n) in einem Raum, beispielsweise in einem Ringspalt gehalten werden, der von der Elektrode zusammen mit einer semipermeablen Membran gebildet wird, oder

(c) sowohl die Peroxidase als auch die Oxidase(n) in einem Raum, beispielsweise in einem Ringspalt, gehalten werden, der von der Elektrode zusammen mit einer semipermeablen Membran gebildet wird.

Dabei können die Peroxidase und gegebenenfalls die Oxidase(n) kovalent an die Elektrode gebunden sein, beispielsweise mit Hilfe von Carbodiimid oder Glutaraldehyd.

Bei dem leitenden Elektrodenmaterial kann es sich um Graphit oder ein Material mit einem Gehalt an Graphit handeln.

Die erfindungsgemäße Enzymelektrode kann streifenförmig ausgebildet sein, wobei sie auf einem flachen Träger - gegebenenfalls zusammen mit einer Gegenelektrode - angeordnet sein kann.

Die Erfindungsgemäße Enzymelektrode eignet sich zur Bestimmung von Oxidasesubstraten oder zur Bestimmung von Enzymaktivität.

## Kurzbeschreibung der Figuren

Weitere Charakteristika der Erfindung werden mit Verweis auf die beiliegenden Figuren beschrieben:

Fig. 1 zeigt schematisch die Seitenansicht des Querschnitts durch eine Elektrode.

Fig. 2 ist eine schematische Seitenansicht des Querschnitts eines elektrochemischen Steifens.

Fig. 3 zeigt graphisch den zeitlichen Verlauf des erzeugten Kathodenstroms einer ARP/GO-Elektrode bei pH 6.01. Elektrodenpotential 130 mV. Die Pfeile weisen auf eine Injektion von 0.6 mM D-Glucose.

Fig. 4 zeigt graphisch die Kalibrationskurve des Steady-State-Stromflusses gegen die Glucosekonzentration einer ARP/GO-Elektrode bei pH 6.01. Elektrodenpotential 130 mV (1), 530 mV (2) und 630 mV (3).

Fig. 5 zeigt graphisch die Kalibrationskurve des Steady-State-Stromflusses gegen die Ethanolkonzentration einer HRP/AO- Elektrode bei pH 7.0. Elektrodenpotential 0 mV, Modifiziersubstanz DMFc. Elektrode mit eingeschlossenen (1) und immobilisierten (2) Enzymen.

Fig. 6 zeigt graphisch die Kalibrationskurve des Steady-State-Stromflusses gegen die Ethanolkonzentration einer HRP/AO-Elektrode bei pH 7.0. Elektrodenpotential -40 mV, Modifiziersubstanz TTF. Erster (1) und zweiter (2) Betriebstag.

Fig. 7 zeigt graphisch die Kalibrationskurve des Spitzenstromflusses gegen die Ethanol- (1) und Cholesterol-Konzentrationen (2) einer HRP/AO- (1) und einer HRP/ChlO-Elektrode (2) bei pH 7.0 (1,2). 0.4 % Thesit (2), Elektrodenpotential -40 mV (1) und 20 mV (2), Vorkonzentrierungszeit 3.6 min (1) und 10 min (2), Modifiziersubstanz TTF.

Fig. 8 zeigt graphisch den Steady-State-Stromfluß gegen pH einer ARP/ChO- (1) und einer ARP/GO-Elektrode (2). Acetatpuffer (Dreiecke), Phosphatpuffer (Ringe), Glycinpuffer (Punkte). Elektrodenpotential 70 mV (1) und 20 mV (2), Konzentrationen: Cholinchlorid 0.08 mM (1) und Glucose 0.6 mM (2).

Fig. 9 zeigt graphisch den Steady-State-Stromfluß gegen pH einer HRP/AO- (1), einer HRP/D-AAO- (2) und einer HRP/CHO-Elektrode (3). Phosphatpuffer (Punkte), Glycinpuffer (Ringe). Elektrodenpotential 20 mV (1) und 0 mV (2,3), Konzentrationen: Methanol 0.33 mM (1), D-Alanin 0.069 mM (2), Cholinchlorid 0.061 mM (3), Modifiziersubstanz DMFc (1-3).

Fig. 10 zeigt graphisch den zeitlichen Verlauf des Elektroden potentials einer ARP/GO- (1′, 2′) und einer ARP-Elektrode (1-4), pH 7.0 (1, 2), 6.01 (1′, 2′, 3), 4.92 (4), Konzentrationen: $H_2O_2$ 1.3 µM (1), 0.32 mM (2-4), Glucose 0.6 mM (1′), 1.8 mM (2′).

Fig. 11 zeigt graphisch die HRP/AO-Elektrodensensitivität für Methanol gegen das Elektrodenpotential bei pH 7.0; Modifiziersubstanzen: TTF (1), DMFc (2), TCNQ (3).

Fig. 12 zeigt graphisch den zeitlichen Verlauf des HRP/D-AAO-Elektroden-Kathodenstroms gegen die Racemaseaktivität (5.5 U (1) und 2.1 U (2)).

Fig. 13 zeigt graphisch die Kalibrationskurve des Steady-State-Stromflusses gegen die Ethanolkonzentration eines elektrochemischen HRP/AO-Streifens bei pH 9.0, Modifiziersubstanz DMFc, Potential -10 mV.

Herstellung der Elektroden:

A) Wie in Fig. 1 gezeigt, enthält eine Enzymelektrode Graphitstäbchen (1) (Durchmesser 10 mm, Ringsdorff-Werke GmbH). An die Enden der Stäbchen (30 mm lang) wurde jeweils ein Kupferdraht (2) mit Epoxy-Silber (3) geklebt. Die Seiten der Elektroden wurden mit einer Polyethylenfolie (4) isoliert. Das andere Ende der Elektroden wurde mit Schmirgelpapier (220 µm) bearbeitet, um ihm eine sphärische Form (Radius 40 mm) (5) oder flache Form (6) zu geben.

B) Im Anschluss an die Vorbehandlung mit Schmirgelpapier wurde die Elektrode gemäß Figur 1B (flache Form) bei Raumtemperatur mit 5 x 50 µl wasserlöslichem Carbodiimid (N-3-(dimethylaminopropyl)-N′-ethylcarbodiimid-hydrochlorid) in 0.1 M K-Phosphatlösung vom pH 4.5 (20 mg/ml) aktiviert. 0.5 h später wurde die Elektrode mit derselben Lösung gewaschen. Danach wurden 20 µl Pilz-Peroxidase (10 mg/ml) und Glucoseoxidase (20 mg/ml) auf die Elektrode getropft. 0.5 h danach wurde die Elektrode mit Phosphatpuffer (pH 7.0) gewaschen und in diesem Puffer im Kühlschrank aufbewahrt.

Die Pilz-Peroxidase/Alkoholoxidase-Elektrode wurde mit doppelter Immobilisation hergestellt. Nachdem die Pilz-Peroxidase wie oben beschrieben fixiert wurde, wurde die Elektrode mit Phosphatpuffer (pH 7.0) gewaschen. Nun wurden 20 µl Alkoholoxidaselösung (5 mg/ml) in Phosphatpuffer, der 2.5 % Glutaraldehyd enthielt, auf die Elektrode aufgetropft und an der Luft getrocknet. Die Elektrode wurde mit obigem Puffer gewaschen und im Kühlschrank aufbewahrt.

Im Fall der Pilz-Peroxidase/Cholinoxidase-Elektroden wurden 20 µl Cholinoxidase (5 mg/ml) in Phosphatpuffer (pH 7.0) mit einer Dialysemembran (25 µm) (7) am sphärischen Ende der Graphitelektrode eingeschlossen, auf der wie oben beschrieben Pilz-Peroxidase kovalent fixiert worden war. Die Membran wurde mit einem Gummiring (8) in Position gehalten. Die Elektrode wurde in Phosphatpuffer (pH 7.0) im Kühlschrank aufbewahrt.

C) Meerrettichperoxidase enthaltende Elektroden wurden auf folgende Weise hergestellt: Nach Vorbehandlung mit Schmirgelpapier wurde die Graphitoberfläche zweimal mit 50 µl Toluol gewaschen und an der Luft getrocknet. Danach wurden 20 µl der toluolischen Mediatorlösung (10 mg/ml) auf die Elektrode

getropft und verdampft. Dieser Schritt wurde zweimal wiederholt. Nach 0.5 h wurden 20 µl Enzymlösung (20 mg/ml Peroxidase und 5 mg/ml Oxidase) in 0.1 M K-Phosphatpuffer (pH 7.0) mit einer Dialysemembran (25 µm) (7) eingeschlossen, die mit einem Gummiring (8) gehalten wurde. Als Oxidasen wurden Alkoholoxidase, Cholinoxidase und D-Aminosäurenoxidase verwendet.

Ferner wurde ein anderer Elektrodentyp hergestellt, indem man Peroxidase (1 mg) und Alkoholoxidase (5 mg) in 0.1 M K-Phosphatpuffer (pH 7.1) auflöste, der 2.5 % Glutaraldehyd enthielt. 20 µl dieser Lösung wurden auf die Elektrode getropft und getrocknet. Nach 1.5 h wurde die Elektrode mit 0.1 M K-Phosphatpuffer (pH 7.0) gewaschen. Im Fall der Cholesteroloxidase-Elektrode wurden 20 µl Peroxidaselösung (10 mg/ml) wie oben beschrieben eingeschlossen. Cholesteroloxidase wurde in löslicher Form benutzt.

D) Elektrochemische Streifen (Fig. 2) wurden aus Celluloseacetat (0.5 mm) (1) hergestellt. Zwei Kohlefolienelektroden (1 mm) (3, 4) wurden mit Silikongummikleber aufgeklebt. Eine Elektrode (3) war zuvor mit Ag aus KAgCN-Lösung (1 %) bedeckt worden, eine andere (4) wurde mit einem Mediator behandelt, und Meerrettichperoxidase und Alkoholoxidase wurden wie oben beschrieben fixiert. Die Streifen wurden trocken an der Luft aufbewahrt.

Messungen:

Elektrodenstrommessungen wurden bei Raumtemperatur in einem standardisierten Drei-Elektroden-Kreis unter Verwendung von 15 ml Puffer durchgeführt. Alle Potentiale wurden gegen eine KCl-gesättigte Ag/AgCl-Elektrode bestimmt. Zur Ermittlung der Cholesterolkonzentration wurden 4 ml Phosphatlösung (pH 7.0) benutzt, die 0.4 % Thesit enthielt und der 0.37 U Cholesteroloxidase in 40 µl desselben Puffers beigegeben worden waren. Die Enzymelektrode wurde 10 min lang ausgeschaltet, dann wieder eingeschaltet, wobei der Stromfluß aufgezeichnet wurde. Die Dämpfungszeit des Potentiostaten betrug 4 s. Die Stromstärke des elektrochemischen Streifens wurde in einem Zwei-Elektroden-Kreis gemessen. Vor Beginn der Messungen wurde Substratlösung (20 - 50 µl) auf die Elektroden (3, 4) (Fig. 2) aufgetropft. Nach Anschluß der Graphitfolienkontakte (2) an einen Potentiostaten wurde der Stromfluß gemessen.

Reagenzien und Pufferlösungen:

Meerrettichperoxidase (HRP) (E.C. 1.11.1.7) (198.8 U/mg) Grade II, Boehringer Mannheim GmbH.
Pilz-Peroxidase (ARP) (E.C. 1.11.1.7) (Arthromyces ramosus, 2110 U/mg) Suntory Ltd.
Alkoholoxidase (AO) (E.C. 1.1.3.13) (Candida boldini, 8.1 U/mg) Boehringer Mannheim GmbH.
Cholinoxidase (ChO) (E.C. 1.1.3.17) (Alcoligenes species, 11 U/mg) Sigma.
D-Aminosäureoxidase (D-AAO) (E.C. 1.4.3.3) (Schweineniere, 14 U/mg) Sigma.
Cholesteroloxidase (ChlO) (E.C. 1.1.3.6) (Pseudomonas species, 40 U/mg) Sigma.
L-Alaninracemase (L-AR) (B. stearothermophilus) GBF.
Tetracyanochinodimethan (TCNQ) - Polysciences Inc.; 1,1′-Dimethylferrocen (DMFc) - Aldrich: Tetrathiafulvalen (TTF) -Aldrich; D-Alanin - Serva; Cholinchlorid, D-Serin, D-Asparaginsäure, D-Lysin, L-Valin, Cholesterol - Sigma; Thesit -Boehringer Mannheim GmbH; N-(3-dimethylaminopropyl)-N′-ethylcarbodiimid-hydrochlorid - Fluka.
Andere Reagenzien - Merck.
Verwendete Puffer: 0.1 M Acetat (pH 4.5 - 5.5), 0.1 M K-Phosphat (pH 5.5 - 8.0) und 0.1 M Glycin (pH 8 - 11.7).
Glucoselösungen wurden über Nacht aufbewahrt, um die Gleichgewichtseinstellung der alpha- und beta-Anomere sicherzustellen. Die Ethanollösungen zur Streifenkalibration enthielten 0.1 M KCl.

Ansprechzeit der Enzymelektrode und Kalibration:

Der Basalstromfluß der ARP/GO-Elektrode in Phosphatpuffer (pH 6.01) war bei einem Elektrodenpotential von 130 mV vernachlässigbar klein. Bei Zugabe der Glucoselösung stieg der Kathodenstrom an. Die Ansprechzeit (90 % des Steady-State-Stroms) betrug 12 s (Fig. 3). Im Konzentrationsbereich von 0.5 bis 6.5 mM Glucose ergab sich eine hyperbolische Kalibrationskurve, $I_{max}$ = 33 µA und $K_{m(app)}$ = 12.5 mM (Fig. 4). Bei Erhöhung des Elektrodenpotentials verringerte sich der Maximalstrom, aber $K_{m(app)}$ änderte sich nicht sehr. Bei einem Elektrodenpotential von 530 und 630 mV betrug $K_{m(app)}$ etwa 11.1 bzw. 9.8 mM. Die

Sensitivität der Elektrode in einem Konzentrationsbereich bis 2 mM Glucose betrug 2.6 mA/M (E = 130 mV), 1.9 mA/M (E = 530 mV) und 1.6 mA/M (E = 630 mV).

Die Bienzym-ARP/AO-Elektrode erzeugte einen Kathodenstrom bei Zugabe von Methanol zum Puffer. Die Ansprechzeit dieser Elektrode, die doppelt immobilisierte Enzyme enthielt, war bis auf 30 s verlängert. Bis zu einer Konzentration von 0.7 mM Methanol zeigte sich eine lineare Korrelation zwischen Elektrodenstrom und eingesetztem Substrat (pH 7.0). Die Sensitivität der Elektrode betrug 0.6 mA/M (E = 30 mV).

Die Ansprechzeit der Bienzym-ARP/ChO-Elektrode war bis auf 40 s verlängert. Diese Elektrode zeigte eine lineare Kalibrationskurve bis 2.5 mM Cholinchlorid (pH 7.0). Die Elektrodensensitivität betrug 0.48 mA/M (E = 70 mV).

Der Basalstromfluß der auf DMFc-modifiziertem Graphit basierenden HRP/AO-Elektrode war bei -30 mV vernachlässigbar klein (pH 7.0). Eine solche Elektrode zeigte bei Einschluß der Enzyme in eine Dialysemembran eine Ansprechzeit von 40 s. Die Kalibrationskurve war bis zu einer Ethanolkonzentration von 1.0 mM linear (Fig. 5). Die auf fixierten Enzymen beruhende Elektrode hingegen zeigte eine hyperbolische Kalibrationskurve;

$$K_{m}(app)$$

betrug 14.3 mM (Fig. 5). Die Ansprechzeit dieser Elektrode betrug 30 s.

Die Kalibrationskurve der TTF-HRP/AO-Elektroden ähnelte denen der DMFc-modifizierten Systeme (Fig. 6). Wenn die HRP/AO-Elektrode im ausgeschaltetem Zustand in die Ethanollösung getaucht und 3.6 min später eingeschaltet wurde, war der Spitzenstrom 25-mal höher als der Steady-State-Strom, und er war bis zu einer Ethanolkonzentration von 0.6 mM linear (Fig. 7).

Die Kalibrationskurven der TCNQ-modifizierten HRP/AO-Elektroden waren denen der DMFc- und TTFmodifizierten Systeme sehr ähnlich. Einen Parametervergleich der HRP/AO-Elektroden gibt Tabelle 1 wieder:

Tab. 1.

| Parameter der HRP/AO-Elektroden | | | | | | |
|---|---|---|---|---|---|---|
| Enzym | Modifiziersubstanz | pH | E (mV) | Substrat | Messbereich in mM | Enzymimmobilisation |
| Alkoholoxidase, Peroxidase | DMFc | 7.0 | 0 | Ethanol | 0.2 - 1.0 | eingeschlossen |
| Alkoholoxidase, Peroxidase | DMFc | 7.0 | 0 | Ethanol | 0.8 - 9.0 | kovalentgebunden |
| Alkoholoxidase, Peroxidase | TTF | 7.0 | -40 | Ethanol | 0.12 - 0.80 | eingeschlossen |
| Alkoholoxidase, Peroxidase | TTF | 7.0 | -130 | Methanol | 0.20 - 0.60 | eingeschlossen |
| Alkoholoxidase, Peroxidase | TCNQ | 7.0 | 0 | Methanol | 0.20 - 0.60 | eingeschlossen |
| D-Aminosäureoxidase, Peroxidase | TTF | 7.5 | -10 | D-Valin | 0.008 - 0.20 | eingeschlossen |
| D-Aminosäureoxidase, Peroxidase | DMFc | 8.0 | 0 | D-Alanin | 0.01 - 0.14 | eingeschlossen |
| Cholinoxidase, Peroxidase | DMFc | 8.5 | 20 | Cholinchlorid | 0.06 - 0.3 | eingeschlossen |
| Peroxidase (lösliche Cholesteroloxidase) | TTF | 7.0 | 20 | Cholesterol | 0.013 - 0.26 | eingeschlossen |

Die Bienzym-HRP/D-AAO-Elektrode zeigte eine lineare Kalibrationskurve bis zu einer Konzentration von 0.2 mM D-Valin. Der Messbereich dieser Substanz lag bei 8 - 200 µM (Tab. 1).

Die Kalibrationskurve der Bienzym-HRP/ChO-Elektrode war bis zu einer Konzentration von 0.3 mM Cholinchlorid linear. Der Messbereich lag bei 0.06 - 0.3 mM (Tab. 1).

Eine HRP/ChlO-Bienzymelektrode, deren Enzyme eingeschlossen oder kovalent gebunden wurden, zeigte zwar eine Reaktion auf Wasserstoffperoxid, aber kein Ansprechen auf Cholesterol. Deshalb wurde zur Cholesterolbestimmung eine lösliche Cholesteroloxidase verwendet. Im Anschluß an die biokatalytische Vorkonzentrierungs- zeit wurde die Elektrode entblößt, um den Elektrodenstrom zu erhöhen und auch um Ethanol zu messen (Fig. 7). Während der Vorkonzentrierungsperiode von 10 min zeigte sich bis zu einer Konzentration von 126 mM eine lineare Beziehung zwischen Maximalstrom und der Cholesterolkonzentration (Fig. 7).

pH-Abhängigkeit des Enzymelektrodenstroms:

Die Bienzym-ARP/GO-Elektrode entwickelte im pH-Bereich von 4.5 - 10.5 einen Kathodenstrom (Fig. 8). Die höchste Aktivität zeigte die Elektrode dabei im pH-Intervall zwischen 5.5 und 8.0 in Phosphatpuffer. Glycin oder Acetat senkten aber den Stromfluß nicht wesentlich.

Das pH-Optimum der ARP/ChO-Elektrode lag bei pH 8.7 (Fig. 8). Demzufolge war die Aktivität in Glycinpuffer höher.

Die HRP/AO-Elektroden zeigten in einem weiten pH-Bereich eine Abhängigkeit der Elektrodensensitivität (Fig. 9). Die aktivste Elektrode hatte ihr Optimum bei pH 10. Die Aktivität war in Glycinpuffer 75 % höher als in K-Phosphatpuffer.

Die HRP/D-AAO-Elektrode entwickelte die größte Aktivität bei pH 8.5 (Fig. 9). Sie war in Glycinpuffer höher als in K-Phosphatpuffer.

Der pH-abhängige Bereich der HRP/ChO-Elektrodenaktivität war im Vergleich zu den HRP/AO- oder HRP/D-AAO-Elektroden zum Alkalischen hin verschoben (Fig. 9). Wiederum war die Signalantwort in Glycinpuffer höher als in Phosphatlösungen.

Potentialabhängigkeit der Enzymelektroden:

Genau wie Monoenzym-ARP-Elektroden bildete die Bienzym-ARP/GO-Elektrode dasselbe positive Potential (Fig. 10). Das Steady-State-Potential der ARP/GO-Elektrode betrug bei pH 6.01 691 mV und 726 mV, wenn 0.6 mM bzw. 1.8 mM Glucose eingesetzt werden. Bei hohen $H_2O_2$-Konzentrationen lagen die Steady-State-Potentiale der ARP-Elektrode bei 682 mV (pH 7.0), 732 mV (pH 6.01) und 769 mV (pH 4.92). Wegen des hohen Potentials erfuhr die Sensitivität der Bienzymelektrode genau wie die der Monoenzym-ARP-Elektrode bei Heraufsetzung des Elektrodenpotentials von 130 mV auf 630 mV keine große Veränderung (Fig. 4).

Die Abhängigkeit der HRP/AO-Elektrodensensitivität vom Elektrodenpotential ist in Fig. 11 dargestellt. Im Bereich um 0.0 V waren jene Elektroden, die mit modifiziertem Graphit arbeiten, die empfindlichsten. Unterschiedlich war dabei jedoch das Potential, bei welchem der Basisstrom am niedrigsten lag. Bei TTFmodifizierten Elektroden war dies bei etwa -130 mV der Fall, bei DMFc-modifizierten Elektroden liegt der Wert um -30 mV und bei TCNQ-Modifizierung um -60 mV. Bei höheren Spannungen steigt der Basisstrom der TTF- und DMFc-modifizierten Elektroden an und die Empfindlichkeit nimmt ab. Im Falle der TCNQ-modifizierten Elektroden blieb der Basisstrom bei positiven Potentialwerten annähernd konstant, aber die Sensitivität wurde kleiner. Für alle modifizierten Elektroden gilt, daß bei Potentialwerten kleiner als den "Null-Basisstrom-Potentialen" der Basalstrom zunahm und die Empfindlichkeit schwand.

Spezifität der Enzymelektroden:

Die HRP/AO- und HRP/D-AAO-Elektroden zeigten eine weitgehende Substratspezifität (Tab. 2).:

Tabelle 2.

| Spezifität der HRP/AO- und HRP/D-AAO-Elektroden | | | |
|---|---|---|---|
| Alkohol-Sensitivität[1] | | D-Aminosäure-Sensitivität[2] | |
| Substrat | relative Sensitivität in % | Substrat | relative Sensitivtät in % |
| Methanol<br>Ethanol<br>1-Propanol<br>2-Propanol | 100<br>28.3<br>12.5<br><1 | D-Valin<br>D-Alanin<br>D-Serin<br>D-Asparaginsäure<br>D-Lysin<br>L-Valin<br>L-Alanin | 100<br>93<br>20<br><1 |
| Messbedingungen: pH 7.0, E = -40 mV, 0.1 M K-Phosphatpuffer, TTF-modifizierte Elektrode (1).<br>pH 7.5, E = -10 mV, 0.1 M K-Phosphatpuffer, TTF-modifizierte Elektrode (2). | | | |

Die HRP/AO-Elektrode gab sowohl auf 1-Propanol wie auch auf Methanol und Ethanol ein Signal. Methanol war am effektivsten, und bei 2-Propanol gab die Elektrode keine Antwort. Die HRP/D-AAO-Elektrode reagierte auf D-Valin und andere hydrophobe D-Aminosäuren (Tab. 2). Wenn L-Alanin durch Alaninracemase umge setzt wurde, steig das Elektrodensignal an; dieser Umstand wurde zur Bestimmung der Racemaseaktivität ausgenutzt (Fig. 12).

Stabilität der Enzymelektroden:

Die untersuchten Elektroden zeigten eine unterschiedliche Langzeitstabilität. Die Aktivität der ARP/GO-Elektroden nimmt jeden Tag etwa um 12 - 15 % ab (bei einer täglichen Meßzeit von - 3 h), und die Restaktivität nach 7 Tagen betrug 30 - 40 % der Ausgangsaktivität. Im Falle der ARP/AO-Elektrode lag der durchschnittliche Aktivitätsverlust nach einem 2-h-Betrieb bei 36 %. Am nächsten Tag fanden wir nur noch 5 - 7 % der Ausgangsaktivität, wobei das Meßsignal auf $H_2O_2$ nur um 16 % abnahm. Dieselbe Stabilität konnte für die HRP/AO-Elektrode beobachtet werden (Fig. 6). Die ARP/ChO-Elektrode zeigte sich stabiler und behielt ihre Effizienz 2 - 3 Tage lang. Während dieser Zeit verminderte sich die Antwort auf $H_2O_2$ auf 60 %.

Die Langzeitstabilität der geprüften Elektroden war hauptsächlich durch die Stabilität der immobilisierten Oxidase festgelegt, da inaktive Elektroden ohne Oxidaseaktivität eine Initialantwort auf Wasserstoffperoxid zeigten.

Die GO enthaltenden Elektroden erwiesen sich als die stabilsten. Dies steht im Einklang mit der hohen Stabilität des nativen Enzyms, ChO und AO waren weniger beständig. Zumindest was das letztere Enzym anbelangt, können die anderen Methoden der Immobilisation hergenommen werden, um es zu stabilisieren.

Kalibration des elektrochemischen Streifens:

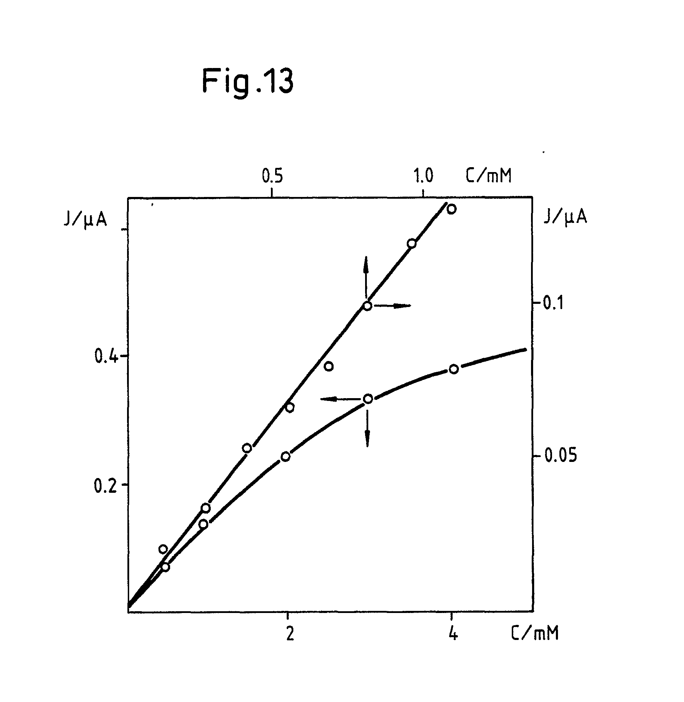

Als Basis zur Herstellung eines elektrochemischen Streifens wurde eine Bienzymelektrode verwendet (Fig. 2). Die Kalibrationskurve der HRP/AO-Streifen war bis zu einer Ethanolkonzentration von 1 mM linear (Fig. 13). Sättigung war bei Ethanolkonzentrationen > 2 mM feststellbar.

Erklärung der Wirkungsweise einer Bienzymelektrode:

Sowohl die auf ARP allein basierenden wie auch die auf ARP und Oxidase beruhenden Elektroden

erzeugen einen Kathodenstrom. Das Steady-State-Potential der ARP- und ARP/GO-Elektroden war extrem hoch. Bei demselben pH-Wert (6.01) ist es für die ARP-Elektrode ($H_2O_2$-Wirkung) und für die ARP/GO-Elektrode (Glucosewirkung) praktisch identisch. Die erwähnten Ergebnisse lassen den Schluß zu, daß das Elektrodenpotential durch die Peroxidasewirkung festgelegt wird. In Bienzymelektroden wird Wasserstoffperoxid in Anwesenheit der Oxidase erzeugt:

$$S + O_2 \xrightarrow{\text{Oxidase}} P + H_2O_2 \qquad (1)$$

$H_2O_2$ wird durch die fixierte Peroxidase reduziert:

$E + H_2O_2 \text{--->} E_1 + H_2O$ (2)

Verbindung I ($E_1$) wird durch mediatorlosen Elektronentransfer zum aktiven Zentrum des Enzyms reduziert:

$E_1 + \ominus \text{--->} E_2$ (3)

$E_2 + \ominus \text{--->} E$ (4)

Diese Annahme wird durch die Tatsache bekräftigt, daß das Potential eines Einzel-Elektronentransfers von Verbindung I ($E_1$) und Verbindung II ($E_2$) dem Steady-State-Potential der Bienzymelektroden sehr nahekommt.

Im Falle der HRP laufen die Reaktionen (3) und (4) langsam ab. Deswegen ist es notwendig, immobilisierte elektrochemisch aktive Verbindungen zu nutzen, die durch HRP oxidiert werden:

$2\,DMFc^{\circ} + H_2O_2 + 2\,H^{+} \text{--->} 2\,DMFc^{+} + 2\,H_2O$ (5)

$2\,TTF^{\circ} + H_2O_2 + 2\,H^{+} \text{--->} 2\,TTF^{+} + 2\,H_2O$ (6)

$2\,TCNQ^{-} + H_2O_2 + 2\,H^{+} \text{--->} 2\,TCNQ + 2\,H_2O$ (7)

Die Reaktionen an der Elektrode sind:

$DMFc^{+} + \theta \text{--->} DMFc$ (8)

$TTF^{+} + \theta \text{--->} TTF$ (9)

$TCNQ + \theta \text{--->} TCNQ^{-}$ (10)

Literaturverzeichnis:


1. Schmid, R.D. et al. (1988). Biosensors and "Bioelectronics". In Biotechnology (Ed. H.-J. Rehm, G. Reed) VCH Verlagsgesellschaft, Weinheim, 6b, 317 - .
2. Kulys, J.J. et al. (1981). Bioelectrochem. Bioenerg., 8, 81 - 88.
3. Shinmen, Y. et al. (1986). Agric. Biol. Chem., 50, 247 -249.
4. Cenas, N.K. et al. (1981). Bioelectrochem. Bioenerg., 8, 103 -113.
5. Prospekt: Neue Peroxidase aus dem Pilz Arthromyces ramosus. Suntory Ltd. Institute for fundamental Research Mischima-gun, Osaka 618 Japan.
6. Aston, W.J. (1987). Biosensors Fundamentals and Application (Ed. A.P.F. Turner, I. Karube, G.S. Wilson), Oxford University Press, Oxford, New York, Tokyo, 276 - 291.
7. Davis, G. (1977). Biosensors Fundamentals and Application (Ed. A.P.F. Turner, I. Karube, G.S. Wilson), Oxford University Press, Oxford, New York, Tokyo, 247 - 257.
8. Hayashi, I. et al. (1979). J. Biol. Chem.,254, 9101 - 9106.


## Ansprüche

1. Enzymelektrode mit einem Bienzymsystem, dadurch *gekennzeichnet,* daß das Bienzymsystem Pilzperoxidase und ein oder mehrere Oxidasen aufweist und frei von Mediatoren ist.
2. Enzymelektrode mit einem Bienzymsystem, dadurch *gekennzeichnet,* daß das Bienzymsystem Meerrettich-Peroxidase, ein oder mehrere Oxidasen und einen wasserunlöslichen Mediator aufweist.
3. Enzymelektrode nach Anspruch 2, *gekennzeichnet* durch Dimethylferrocen oder Tetrathiafulvalen als Mediator.
4. Enzymelektrode nach einem der Ansprüche 1 bis 3, dadurch *gekennzeichnet,* daß das Bienzymsystem als Oxidase Glukoseoxidase, Alkoholoxidase, Cholinoxidase, D-Aminosäureoxidase und/oder Cholesteroloxidase aufweist.

5. Enzymelektrode nach einem der vorhergehenden Ansprüche, dadurch *gekennzeichnet,* daß

(a) die Peroxidase und die Oxidase(n) aur der Elektrode immobilisiert sind oder

(b) die Peroxidase auf der Elektrode immobilisiert ist und die Oxidase(n) in einem Raum gehalten werden, der von der Elektrode mit einer semipermeablen Membran gebildet wird, oder

(c) daß sowohl die Peroxidase als auch die Oxidase(n) in einem Raum gehalten werden, der von der Elektrode mit einer semipermeablen Membran gebildet wird.

6. Enzymelektrode nach Anspruch 5, dadurch *gekennzeichnet,* daß die Peroxidase und gegebenenfalls die Oxidase(n) kovalent an die Elektrode gebunden sind.

7. Enzymelektrode nach einem der vorhergehenden Ansprüche, dadurch *gekennzeichnet,* daß das leitende Elektrodenmaterial Graphit umfaßt.

8. Enzymelektrode nach einem der vorhergehenden Ansprüche, dadurch *gekennzeichnet,* daß sie streifenförmig ausgebildet ist.

9. Enzymelektrode nach einem der vorhergehenden Ansprüche, dadurch *gekennzeichnet,* daß sie auf einem flachen Träger gegebenenfalls zusammen mit einer Gegenelektrode vorgesehen ist.

10. Verwendung einer Enzymelektrode gemäß einem der vorhergehenden Ansprüche zur Bestimmung von Oxidasesubstraten oder einer Enzymaktivität.

Fig.1

(A)

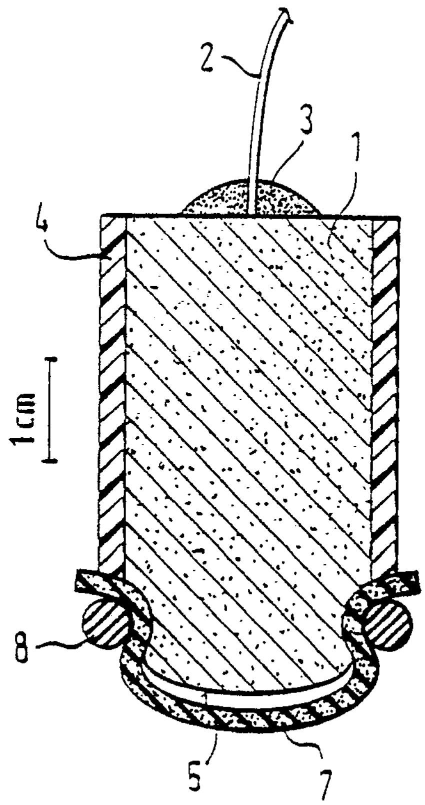


(B)

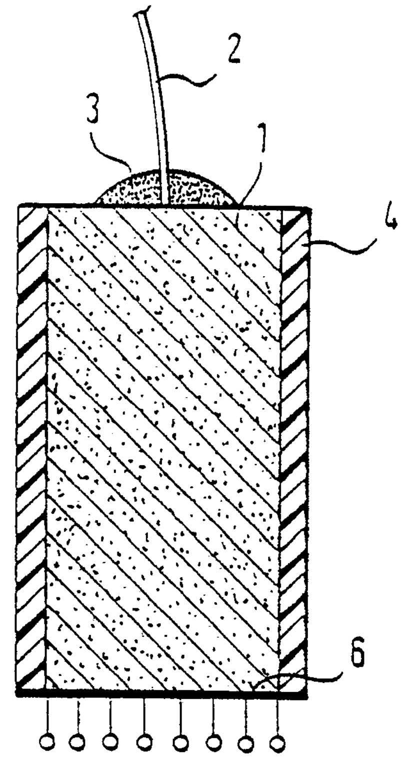


Fig.2

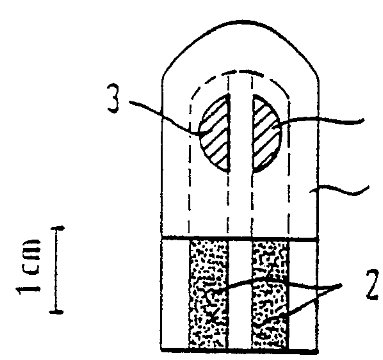

Fig.3

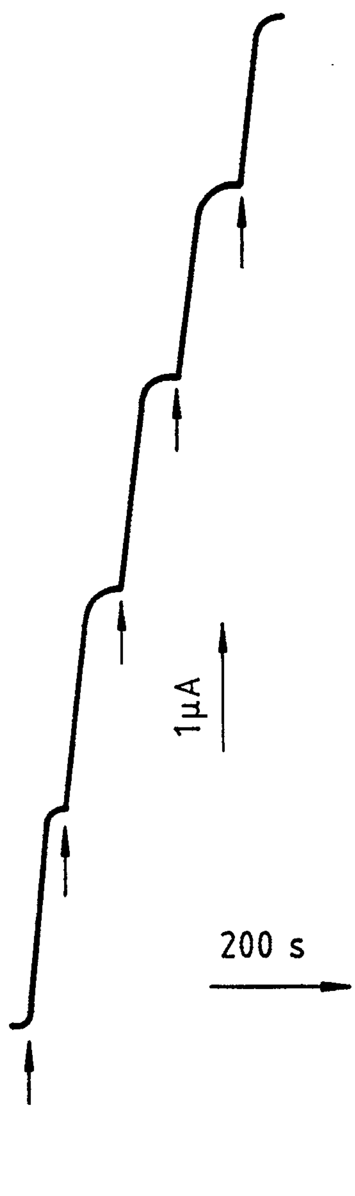

Fig. 4

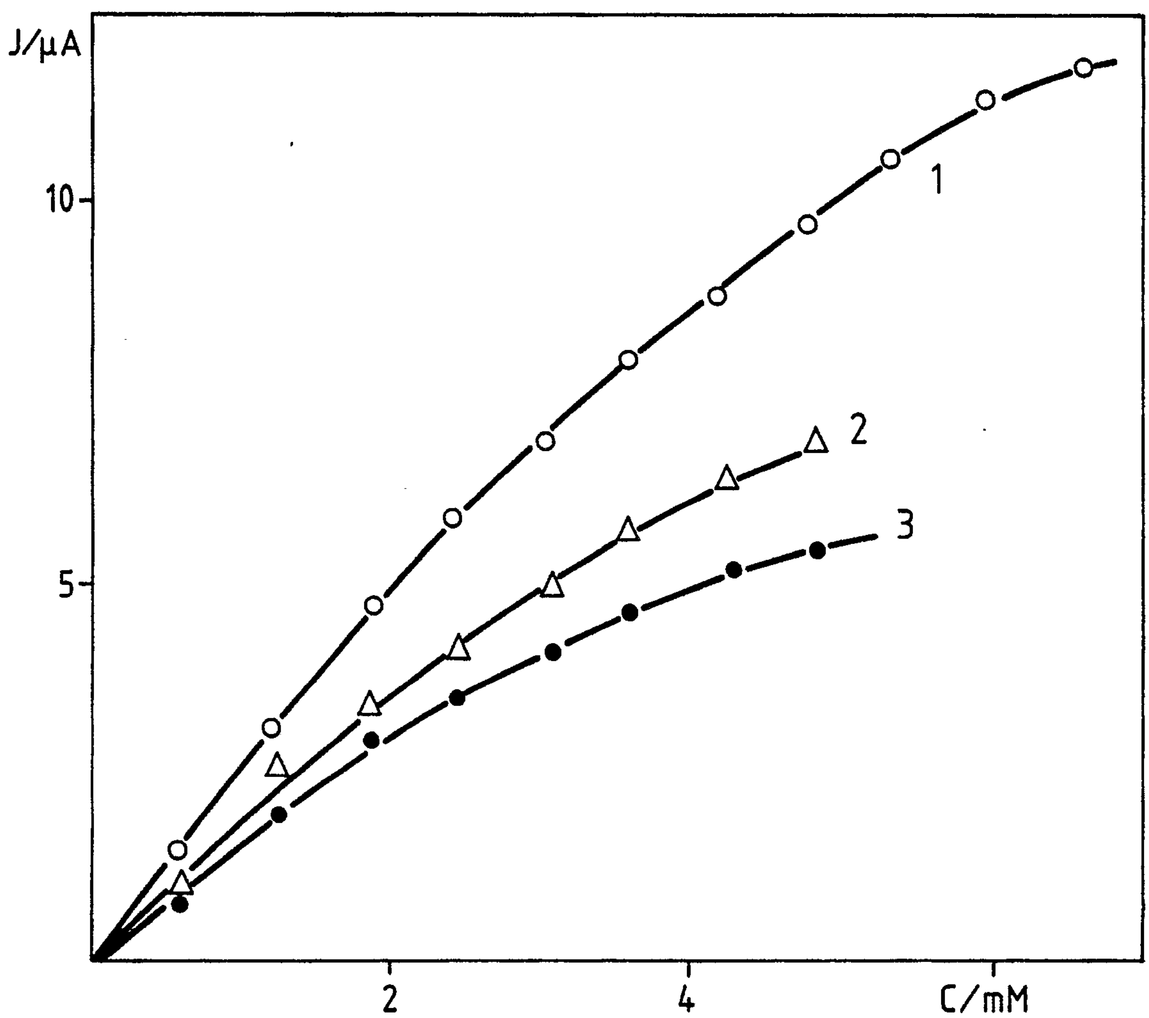

Fig.5

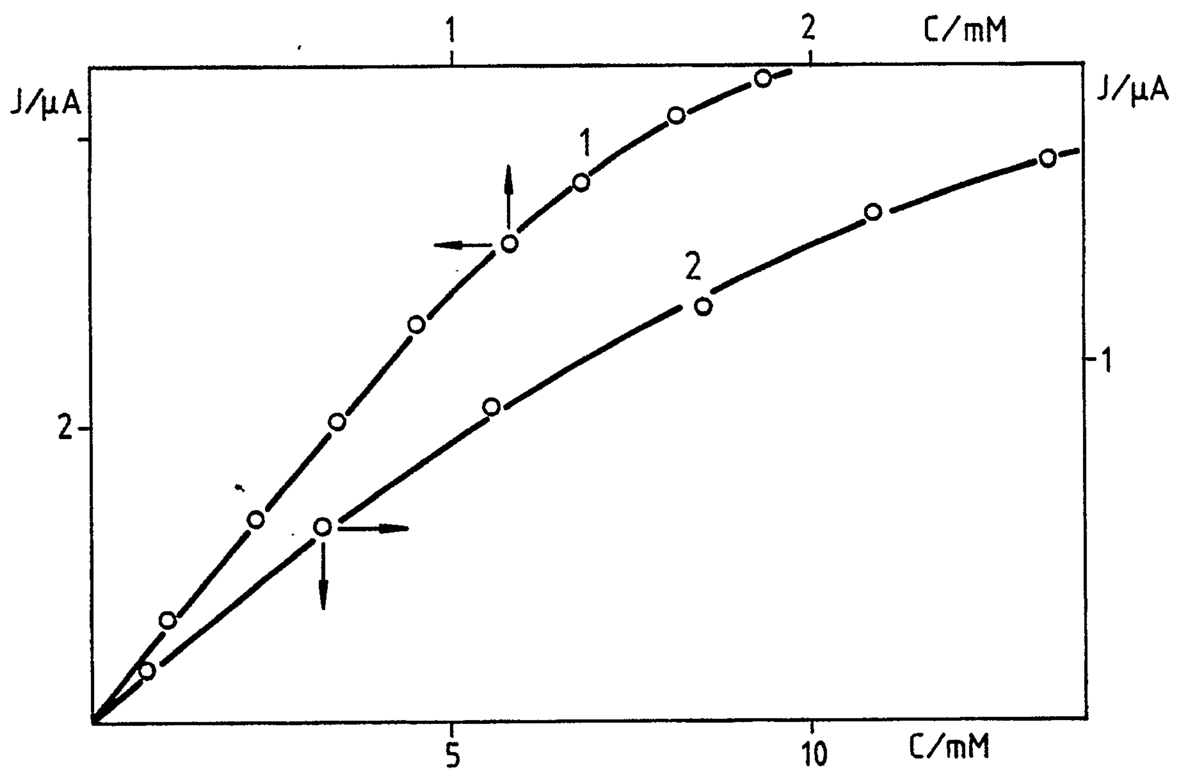

Fig.6

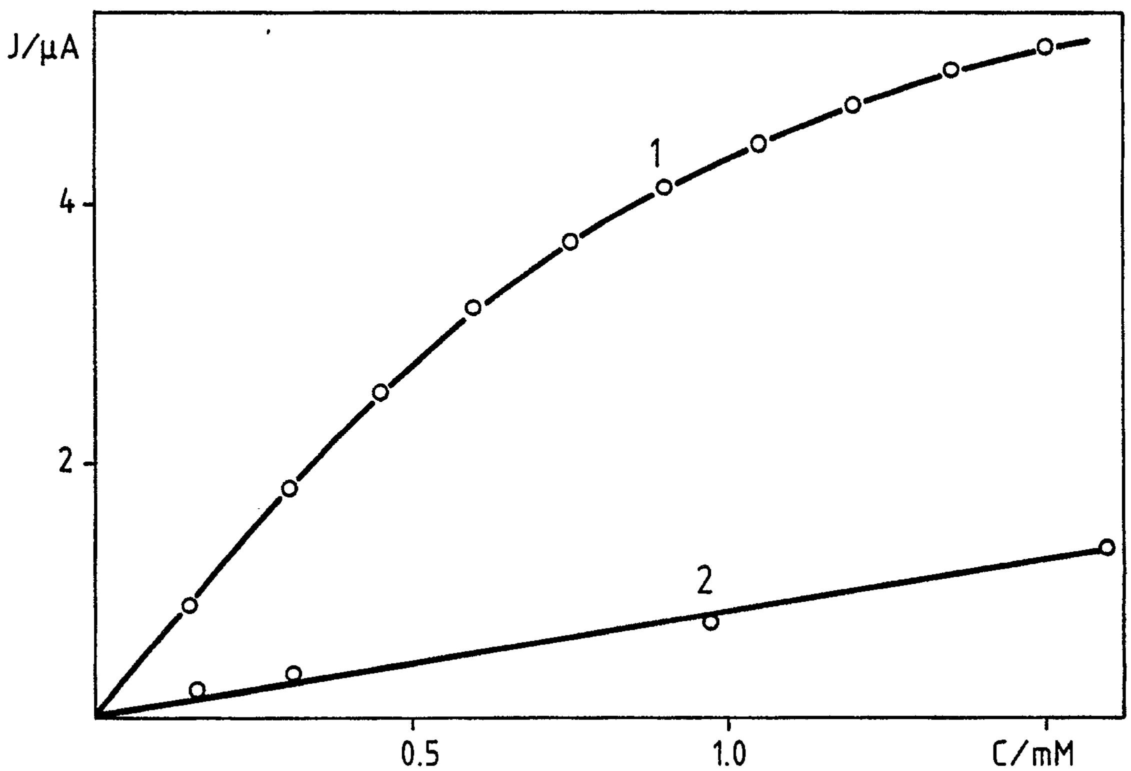

Fig.7

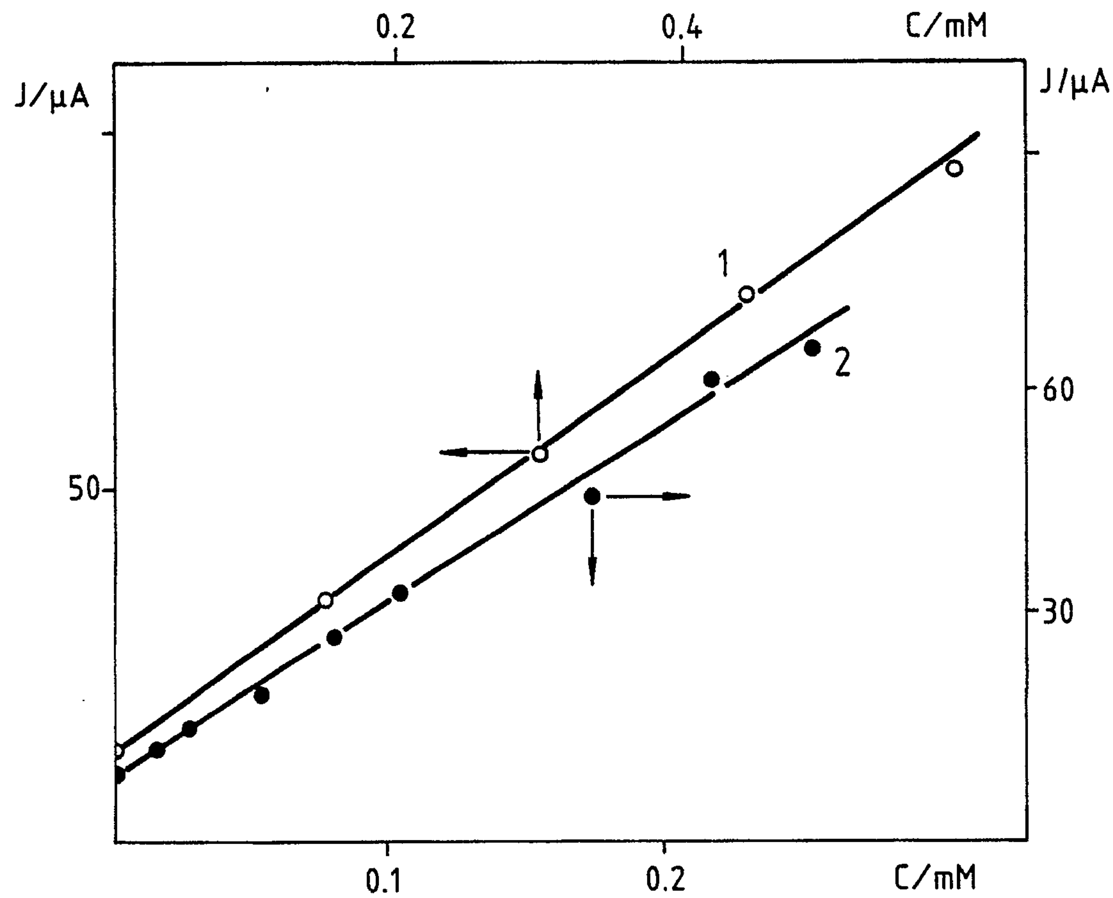

Fig. 8

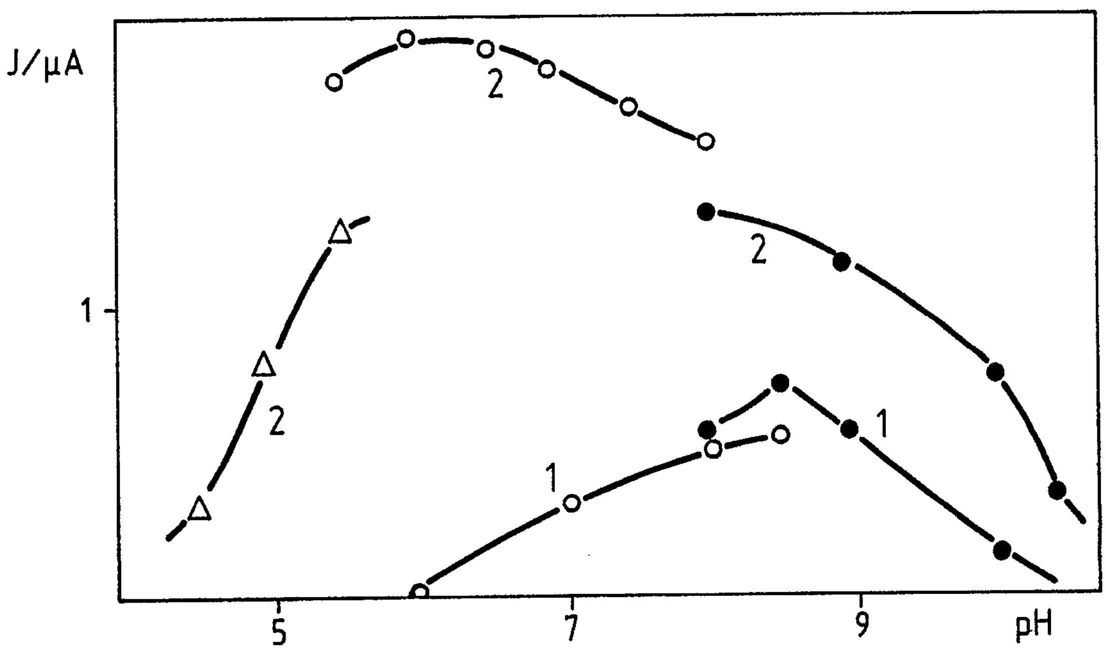

Fig. 9

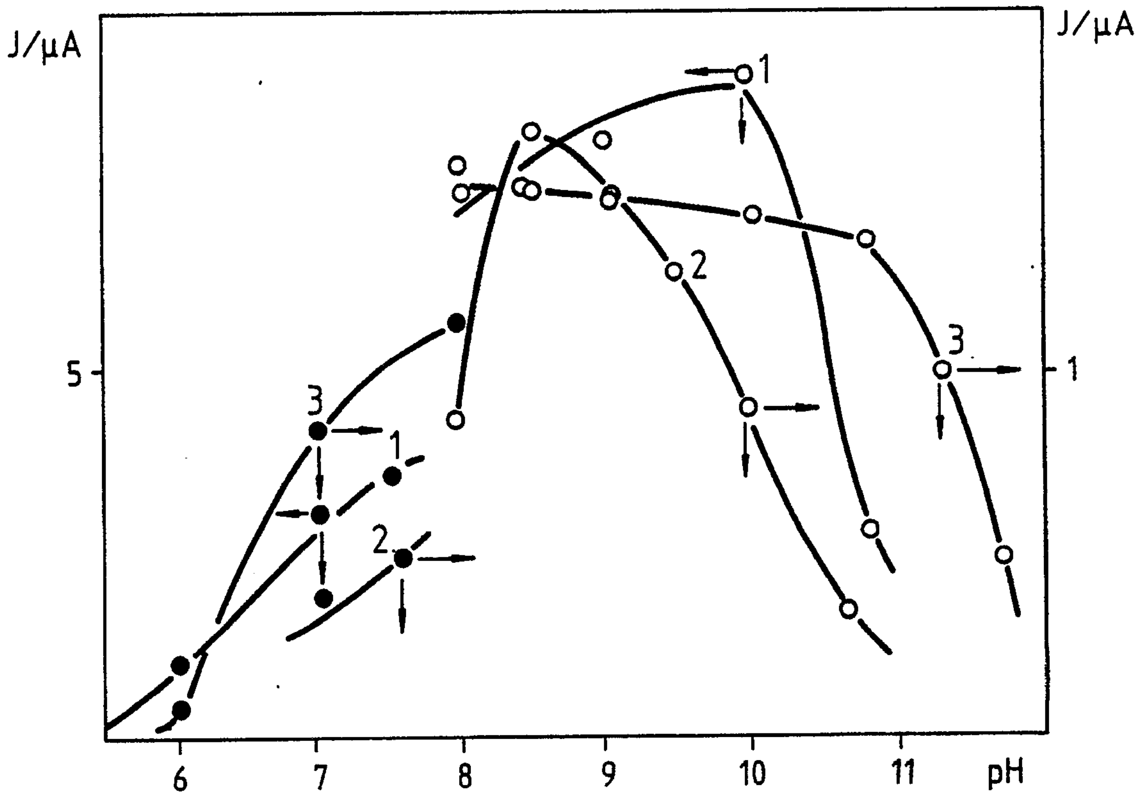

Fig.10

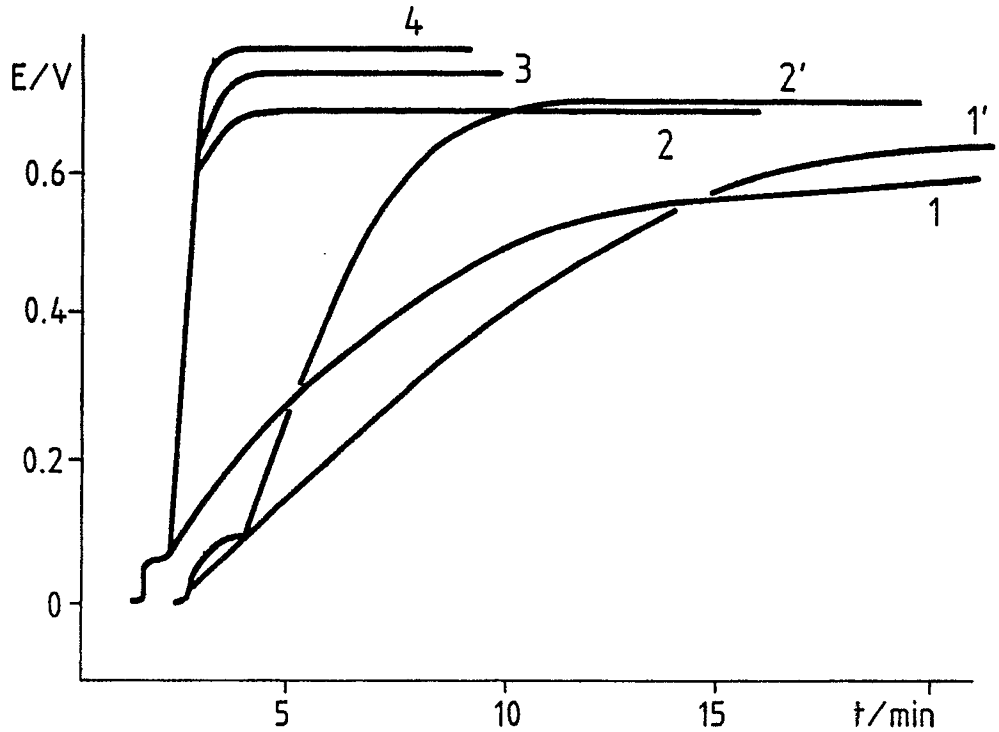

Fig.11

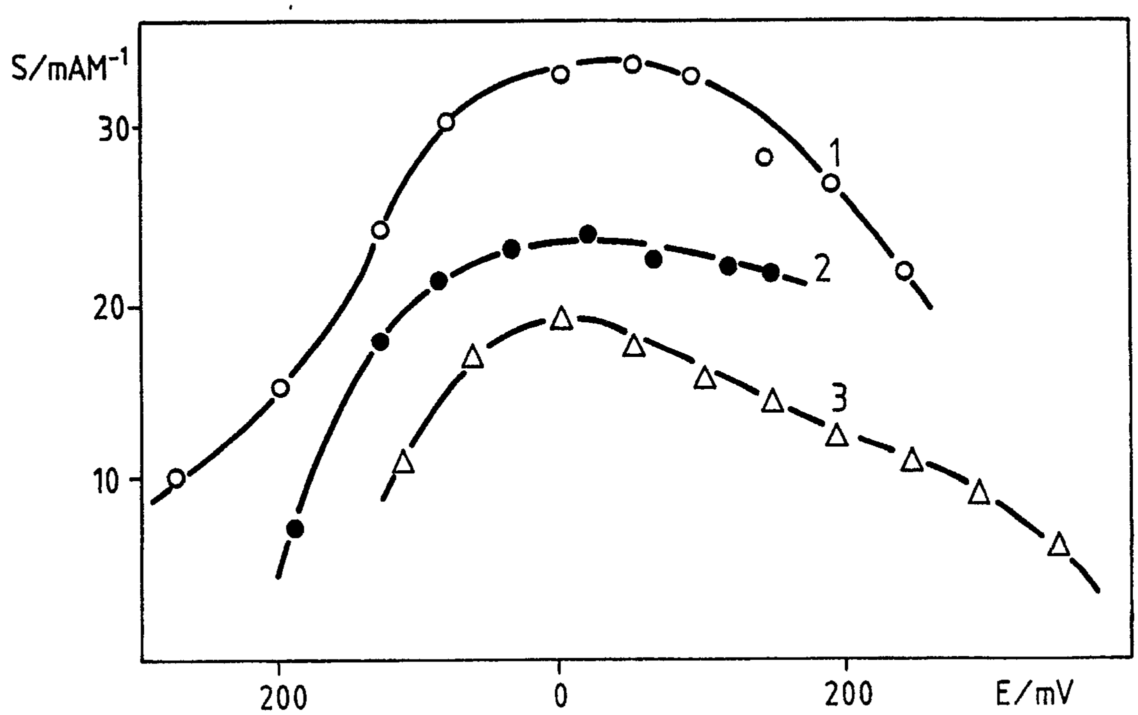

Fig.12

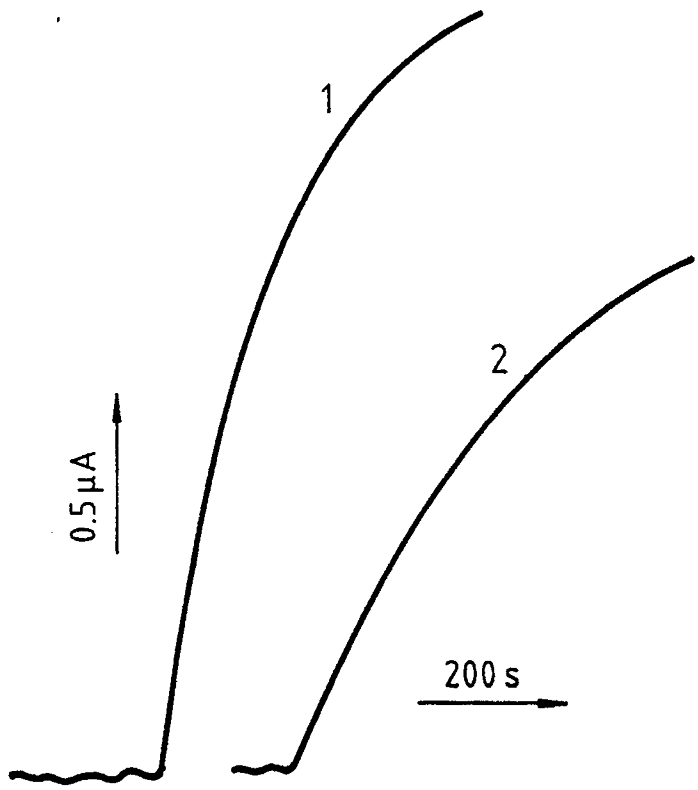

Fig.13